# EUROPEAN PATENT SPECIFICATION

(11) **EP 3 442 464 B1**
(45) Date of publication and mention of the grant of the patent: **14.10.2020**
(21) Application number: 17718466.0
(22) Date of filing: 11.04.2017
(51) Int. Cl.: A61C 13/15, A61B 1/06, A61B 1/24

(54) **A DENTAL LIGHT POLYMERIZATION DEVICE**
ZAHNÄRZTLICHE LICHTPOLYMERISIERUNGSVORRICHTUNG
DISPOSITIF DENTAIRE DE POLYMÉRISATION À BASE DE LUMIÈRE

(30) Priority: 15.04.2016 EP 16165494
(43) Date of publication of application: 20.02.2019
(73) Proprietor: 3M Innovative Properties Company, St. Paul, MN 55133-3427 (US)
(72) Inventor: SCHMID, Rudolf, 82229 Seefeld (DE); GERLACH, Korbinian, 82229 Seefeld (DE); WELKER, Stefan K., 82229 Seefeld (DE); KELZ, Ralf, 82229 Seefeld (DE); MÜLLER, Thomas K., 82229 Seefeld (DE)
(74) Representative: Bergen, Katja
(86) International application number: PCT/US2017/026890
(87) International publication number: WO 2017/180547

(56) References cited:
- WO-A1-2014/043488
- JP-A- 2008 086 554
- US-A1- 2005 003 323

## Description

### Field of the Invention

The invention relates to a dental light polymerization device, and in particular tor a dental light polymerization device having a polymerization light source, a camera and an actively movable light reflector.

### Background Art

Light hardenable or light curable materials are widely used in dentistry for the restoration of teeth. Many of such materials are made to provide optical characteristics that resemble those of natural teeth. Further, such materials typically can be placed precisely and conveniently before they are hardened in place instantly. These materials are often preferred alternatives to less pleasant looking and self-hardening materials, like for example amalgam.

Light hardenable materials often include a polymerizable matrix material and filler materials including colorants, and may initially be generally soft or flowable so that they can be applied in a desired location and shape. For example, for restoration of a tooth the dental material may be filled into a tooth cavity and shaped so that the restored tooth resembles a natural tooth. Once the desired shape has been formed, the material may be cured by exposing it to light of a desired wavelength. The light typically activates photoinitiators in the dental material that cause the matrix material to polymerize.

The use of dental materials that are hardenable by blue light of a wavelength of between about 450 and 500 nm (nanometers) has become common in dentistry. Accordingly, light-emitting devices used for hardening such dental materials typically emit light at such wavelengths. Such a light-emitting device is for example available from 3M Deutschland GmbH, Germany, under the trade designation Elipar™ S10.

A variety of light devices have been developed or proposed. Further, light devices having additional functionality have been developed recently. For example WO 2014/043488 A1 discloses a dental irradiation device which has a first light emitting unit for emitting blue light for light hardening of a dental material. The device has a second light emitting unit and an image sensing unit which are adapted for cooperation with each other for simultaneous illumination and image capturing.

Although there are a variety of light devices on the market there is still a desire to provide a device that provides a variety of functions and which is relatively convenient in handling. Further, such a device is desirably inexpensive.

### Summary of the Invention

The invention relates to a dental light polymerization device which comprises a polymerization light source, and a camera. The device further comprises an actively movable light reflector.

The invention is advantageous in that it allows for polymerization of a dental material and capturing of one or more images during the polymerization with the same device. Further, due to the movable mirror the two functions of light emission for polymerization and image capturing can be alternatively activated. Thus, each function can be activated generally at full performance level. Accordingly, the light for polymerization can be used at generally its full intensity while the camera is available for image capturing.

According to the invention, the reflector is movable between a first positional arrangement, in which the reflector establishes a first optical path between the polymerization light source and an object, and a second positional arrangement, in which the reflector establishes a second optical path between the object and the camera. This means that (if no object is present) in the first positional arrangement the reflector establishes the first optical path between the polymerization light source and an imaginary point outside the device, and in the second positional arrangement the reflector establishes the second optical path between the same point and the camera. Preferably, in the first positional arrangement the reflector does not establish the second optical path. Further, in the second positional arrangement the reflector does not establish the first optical path between the object and the camera.

An actively movable light reflector as referred to herein may comprise a motor for driving the reflector, for example on or more mirrors. For example, a micro motor may be provided which carries the reflector. Several techniques of providing a motor are available based on electrically controlled magnetic or electrostatic fields. For example, actively movable light reflector may be formed by a so-called Digital Mirror Device (DMD). Such a DMD comprises one or more (typically a multiplicity) of micro-mirrors which are individually movable by the force of electrostatic fields between two positions.

In the first positional arrangement the second optical path is suspended or interrupted. Further, in the second positional arrangement the first optical path is suspended or interrupted. Furthermore, in the first positional arrangement the second optical path is suspended or interrupted and in the second positional arrangement the first optical path is suspended or interrupted. The suspension or interruption may be caused by arranging the reflector in the first or second optical path so that the respective optical path is interrupted. Alternatively, the first and/or second optical path may be established by the reflector deflecting light along the respective path so that by removing the reflector the light no longer travels on that path.

In one embodiment in the first positional arrangement the reflector establishes the first optical path in that the reflector is arranged outside the first optical path. In this embodiment the first optical path is linear. In this embodiment in the second positional arrangement the reflector establishes the second optical path in that the reflector bends the second optical path. Accordingly the second optical path is bent or angled. In the first positional arrangement according to this embodiment the reflector may be also arranged such that the reflector does not establish the second optical path. Further, in the second positional arrangement according to this embodiment the reflector may be arranged within the first optical and thus interrupts the first optical path.

In a further embodiment the reflector establishes the first optical path and the second optical path in that the reflector alternatively bends both, the first and second optical path. Accordingly, the first and second optical path of this embodiment each are bent or angled. In the first positional arrangement according to this embodiment the reflector may be also arranged such that the reflector does not establish the second optical path, and in the second positional arrangement according to this embodiment the reflector may be arranged such that the reflector does not establish the first optical path.

In a further embodiment the reflector is formed by a prism. For example the reflector may be formed by a mirror that is formed by a mirrored surface of the prism. Further, the reflector may be formed by any mirror. The mirror is preferably planar.

In one embodiment the reflector is rotatable between the first and the second positional arrangement. For example the reflector may be continuously rotatable over 360 degrees. Accordingly, the mirror positions successively at the first and second positional arrangement (and optionally further intermediate positions). Further, the mirror may position continuously and successively at the first and second positional arrangement during a time period that is determined for polymerization of a dental material. Such time period may be a few second up to several minutes.

In a further embodiment the dental light polymerization device is operable for periodically moving the reflector between the first and second positional arrangement. For example, the reflector may be continuously rotatable or pivotable by an angle of less than 360 degrees. One period or cycle may comprise maintaining the reflector in the first positional arrangement for a first time period, moving the reflector to the second positional arrangement, maintaining the reflector in the second positional arrangement for a second time period, and moving the reflector to the first positional arrangement. The first and second time periods are different, preferably at a ratio of between about 40:1 and about 2:1, more preferably at a ratio of between about 20:1 and about 10:1.

In one embodiment the reflector is formed by a multiplicity of micromirrors provided by a Digital Micromirror Device. Such a device is for example available from Texas Instruments Inc., Dallas, TX, USA.

In one embodiment in the first positional arrangement the camera is deactivated, and in the second positional arrangement the polymerization light source is deactivated. In particular, the dental light polymerization device may have a control unit which is configured to activate (switch on) and deactivate (switch off) the polymerization light source in coordination with the mirror being arranged in the first and second positional arrangement, respectively. Further, the control unit may be configured to activate (switch on) and deactivate (switch off) the camera in coordination with the mirror being arranged in the second and first positional arrangement, respectively. Further in the second positional arrangement an illumination light source may be activated. The illumination light source may be deactivated in the first positional arrangement. The activation and deactivation of the illumination light source is preferably also controlled by the control unit of the dental light polymerization device.

In a further embodiment the illumination light source is configured to emit visible light having a spectrum comprising light at wavelengths of between 380 nm and 750 nm. The illumination light source comprises a plurality of white LEDs.

In still a further embodiment the polymerization light source is configured to emit visible light predominantly within a wavelength range 450 nanometers - 495 nanometers. The polymerization light source preferably consists of a single high power LED.

Preferably, the dental light polymerization device has a rechargeable battery. Further, the battery, the polymerization light source, the camera, the illumination light source and the reflector are preferably encapsulated in a closed housing. The housing has at least partially a transparent wall for permitting the polymerization light source and the illumination light source to emit light from the inside of the housing toward an outside point or area. The transparent wall or wall portion further preferably allows for the camera to receive an image from the outer point or area. The housing is preferably shaped so that a portion of the housing fits into the oral cavity of a patient. Preferably, that portion comprises the transparent wall or wall portion.

### Brief Description of the Figures

- Fig. 1: is a concept view of a dental light polymerization device according to an embodiment of the invention at one stage of operation;
- Fig. 2: is a concept view of the dental light polymerization device of Fig. 1 at a different stage of operation;
- Fig. 3: is a concept view of a further dental light polymerization device according to an embodiment of the invention;
- Fig. 4: is a concept view of a further dental light polymerization device according to an embodiment of the invention;
- Fig. 5: is a perspective partial view of a dental light polymerization device according to an embodiment of the invention; and
- Fig. 6: is a perspective partial view of a dental light polymerization device according to another embodiment of the invention.

### Detailed Description of the Invention

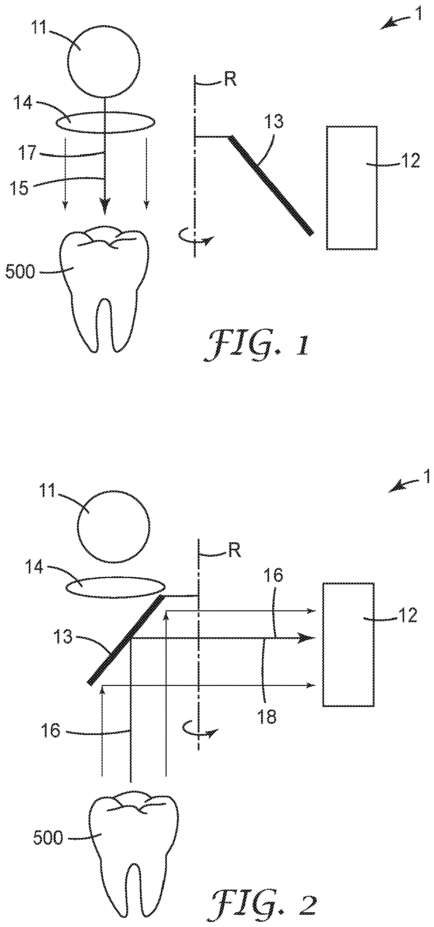

Figures 1 and 2 show an example of a dental light polymerization device 1 of the invention at two different stages of operation. The dental light polymerization device 1 has a polymerization light source 11 and a camera 12. The polymerization light source 11 comprises a blue power LED which emits light in a wavelength range of about 460 nanometers to 490 nanometers and having a maximum light intensity at about 470 nanometers. A blue power LED as it may be used with the present invention is for example available under the designation LUXEON Z Blue from Philips Lumileds Lighting Company. The camera 12 in the example is a color CCD camera. The dental light polymerization device 1 has a mirror 13 which is movable between a first positional arrangement as illustrated in Fig. 1 and a second positional arrangement as illustrated in Fig. 2. In the example the mirror 13 is rotatable about a rotation axis R. The skilled person will however recognize configurations in which the mirror is movable linearly or otherwise as appropriate.

In Fig. 1 (in the first positional arrangement) the mirror 13 establishes a first optical path 15 between the polymerization light source 11 and an object 500 (preferably a tooth in a patient's mouth). In the example the first optical path is established in that the mirror 13 is positioned outside that first optical path 15. Hence, in the example, there is no relevant interaction between the mirror and the polymerization light source 11. The dental light polymerization device 1 further has a lens 14 which is arranged down-beam of the polymerization light source 11. The lens 14 is configured to convert light emitted from the polymerization light source into a generally parallel and generally uniform light beam.

In operation of the dental light polymerization device 1 in the first positional arrangement (Fig. 1) the polymerization light source emits blue light toward the object 500. If the object is a tooth, thus a light hardenable dental material filled in a cavity of that tooth may be hardened by exposure to the blue light emitted by the dental light polymerization device 1.

In Fig. 2 (in the second positional arrangement) the mirror 13 establishes a second optical path 16 between the object 500 and the camera 12. In the example, the second optical path 16 is established in that the mirror 13 is positioned within the first optical path 15, thus blocking light emitted by the polymerization light source 11 from reaching the object, and in that the mirror is positioned such that light coming from the object is deflected by the mirror toward the camera 12.

In operation of the dental light polymerization device 1 the mirror 13 continuously rotates about the rotation axis R and thus alternately changes between the first and second positional arrangement. It is noted that the first positional arrangement of the mirror in this example includes at least a first range of different particular angular positions in which the mirror 13 is positioned outside the first optical path 15. Fig. 1 illustrates one particular position of the mirror 13. There may further be a second range of different particular angular positions in which the mirror 13 establishes the second positional arrangement. However, the second range is smaller, in particular significantly smaller, than the first range. Therefore, in a continuous and uniform rotation the mirror 13 is positioned predominantly in the first positional arrangement and less dominantly in the second positional arrangement. Accordingly, in operation the dental light polymerization device 1 predominantly emits blue light and intermittently captures one or more images from the object while the object is not illuminated by blue light.

As illustrated the polymerization light source 11 is arranged in the dental light polymerization device 1 to emit light in a light emitting direction 17 (along optical path 15) and the camera 12 is arranged in the dental light polymerization device 1 to receive light from an image receiving direction 18 which is transverse or inclined to light emitting direction. The light emitting direction 17 and the image receiving direction 18 in the example intersect outside the object.

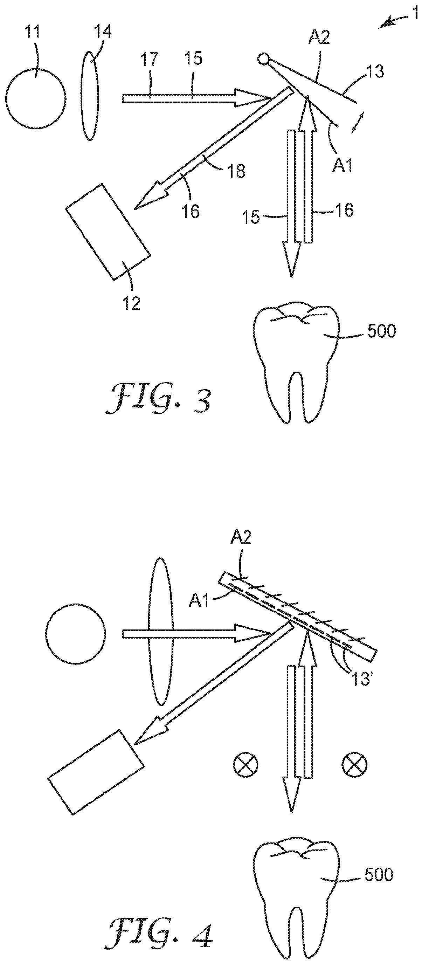

Fig. 3 shows a further example of a dental light polymerization device 1 of the invention. The light polymerization device 1 has a polymerization light source 11 and a camera 12. The polymerization light source 11 and the camera are identical to the example shown in Figures 1 and 2. However, the polymerization light source 11 and the camera 12 are arranged differently with respect to each other compared to the example shown in Figures 1 and 2. In particular, the polymerization light source 11 is arranged such that the light emitting direction 17 of the polymerization light source 11 is directed toward a movable, in particular tiltable, mirror 13. The mirror 13 is movable between a first positional arrangement A1 and a second positional arrangement A2. (The same mirror 13 is shown in the two different first and second positional arrangement.) In the first positional arrangement A1 the mirror 13 establishes a first optical path 15 between the polymerization light source 11 and the object 500. In the second positional arrangement A2 the mirror 13 establishes a second optical path 16 between the object 500 and the camera 12. In this example, in the first positional arrangement A1 the second optical path 16 is disabled. This means that light coming from the object 500 is deflected relative to the second optical path 16. Thus, the light from the object 500 does not reach the camera 500. Further, in the second positional arrangement A2 the first optical path 16 is disabled. This means that light emitted from the polymerization light source 11 is deflected relative to the first optical path 15. Thus, the light from the polymerization light source 11 does not reach the object 500. In the example, the mirror 13 is moved only for the positioning between the first and second positional arrangement A1, A2 and otherwise stands still. The time at which the mirror 13 remains in the first positional arrangement A1 is preferably longer than the time the mirror remains in the second positional arrangement A2. Further, the time for moving the mirror 13 is preferably shorter than the time at which the mirror 13 remains in the first positional arrangement A1, and may be shorter than the time the mirror remains in the second positional arrangement A2.

Fig. 4 shows a further example of a dental light polymerization device 1 of the invention. The dental light polymerization device 1 is identical to the dental light polymerization device 1 shown in Fig. 3, but with the mirror being replaced by a so-called Digital Mirror Device (DMD). A DMD comprises a multiplicity of micro-mirrors 13' which are individually movable by the force of electrostatic fields between two positions. In the example, the micro-mirrors 13' of the DLD are preferably controlled to switch all at a time between a first and a second positional arrangement A1, A2. Again, in the first positional arrangement A1 the micro-mirrors 13' establish a first optical path 15 between the polymerization light source 11 and the object 500. In the second positional arrangement A2 the mirror 13 establishes a second optical path 16 between the object 500 and the camera 12.

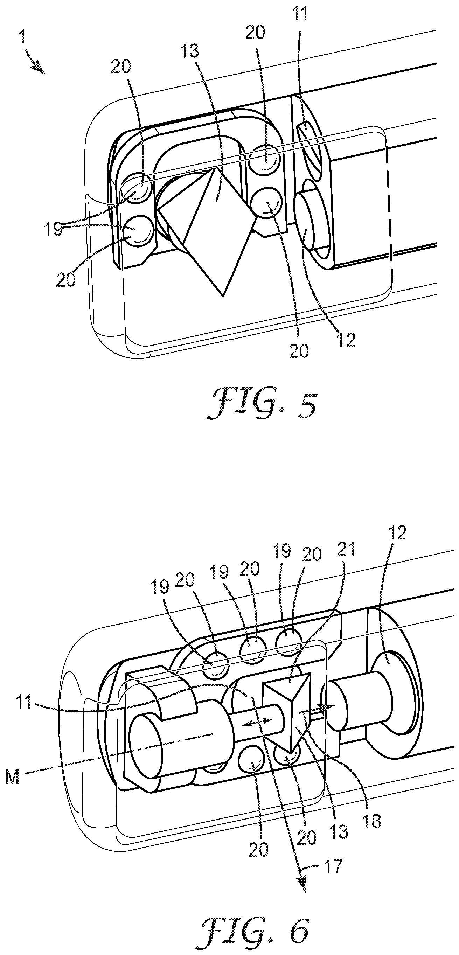

Fig. 5 shows yet another example of a dental light polymerization device 1 of the invention. The dental light polymerization device 1 has a polymerization light source 11 and a camera 12 which is identical with the examples in Figures 1 to 4. A rotatable mirror 13 is arranged so as to establish a first optical path (not illustrated) between the polymerization light source 11 and an object (not shown) and, alternatively, a second optical path between the object and the camera 12. The function of the light polymerization device 1 corresponds to the function as described in the example of Fig. 3.

The light polymerization device 1 of Fig. 5 further has an illumination light source 19, which in the example is formed by a number of individual LEDs 20 which are configured to emit white light. The dental light polymerization device 1 is configured such that the camera 12 and the illumination light source 19 are activated at least temporarily simultaneously during a stage at which the mirror 13 is positioned in the second positional arrangement. Thus, the object can be illuminated by the illumination light source 19 during the camera 12 captures an image of the object. Further, the dental light polymerization device 1 is configured such that the polymerization light source 11 is inactive during the stage at which the mirror 13 is positioned in the second positional arrangement. Thus, light of the polymerization light source 11 does not interfere with the light used for capturing the image of the object. The dental light polymerization device 1 is further preferably configured such that the illumination light source 19 and the camera 12 are inactive during a stage at which the mirror 13 is positioned in the first positional arrangement. In this stage, the polymerization light source 11 is activated.

Fig. 6 shows an example of a dental light polymerization device 1 of the invention. The dental light polymerization device 1 has a polymerization light source 11 and a camera 12 which is identical with the examples in Figures 1 to 4. In this example the dental light polymerization device 1 has a mirror 13 that is linearly movable about a moving axis M. The moving axis M is parallel, in particular coincident, with an image receiving direction 18 of the camera 12. In the example, the mirror 13 is provided by a mirrored surface of a transparent prism 21. The skilled person will however recognize other solutions for providing a mirror, for example a flat mirror which is arranged inclined. The prism 21 and thus the mirror 13 are movable between a first positional arrangement and a second positional arrangement (as shown in the Figure). In the second positional arrangement the mirror 13 is arranged to establish a second optical path between an object (not shown) and the camera 12. Further, in the first positional arrangement the mirror 13 is arranged to establish a first optical path between the polymerization light source 11 and the object (not shown). In the second positional arrangement the prism 21 overlaps a light output area of the polymerization light source 11. Thus, in the second positional arrangement the mirror 13 is positioned within the first optical path, whereas in the first positional arrangement the mirror 13 is arranged outside the first optical path.

The light polymerization device 1 of Fig. 6 further has an illumination light source 19, which in the example is formed by a number of individual LEDs 20. Each of the LEDs 20 are configured to emit white light. The dental light polymerization device 1 is configured such that the camera 12 and the illumination light source 19 are activated at least temporarily simultaneously during a stage at which the mirror 13 is positioned in the second positional arrangement. Further, the dental light polymerization device 1 is configured such that the polymerization light source 11 is inactive during the stage at which the mirror 13 is positioned in the second positional arrangement. The dental light polymerization device 1 is further preferably configured such that the illumination light source 19 and the camera 12 are inactive during a stage at which the mirror 13 is positioned in the first positional arrangement. In this stage, the polymerization light source 11 is activated.

## Claims

1. A dental light polymerization device (1), comprising a polymerization light source (11), and a camera (12), wherein the device comprises an actively movable light reflector,
**characterized in that** the reflector is movable between a first positional arrangement (Fig. 1), in which the reflector establishes a first optical path (15) between the polymerization light source (11) and an object (500), and a second positional arrangement, in which the reflector establishes a second optical path (16) between the object (500) and the camera (12), wherein alternatively :
in the first positional arrangement the second optical path (16) is suspended;
in the second positional arrangement the first optical path (15) is suspended.

2. The dental light polymerization device of claim 1, wherein the reflector is formed by a prism (21).

3. The dental light polymerization device of claim 1, wherein the reflector is formed by a mirror (13).

4. The dental light polymerization device of any of the preceding claims, wherein the reflector is rotatable between the first and the second positional arrangement.

5. The dental light polymerization device of any of the preceding claims, being operable for periodically moving the reflector between the first and second positional arrangement.

6. The dental light polymerization device of claim 5, wherein one period comprises maintaining the reflector in the first positional arrangement for a first time period, moving the reflector to the second positional arrangement, maintaining the reflector in the second positional arrangement for a second time period, and moving the reflector to the first positional arrangement.

7. The dental light polymerization device of claim 6, wherein the first and second time periods are different, preferably at a ratio of about 40:1 to about 2:1.

8. The dental light polymerization device of claim 3, wherein the reflector is formed by a multiplicity of micromirrors (13') provided by a Digital Micromirror Device.

9. The dental light polymerization device of any of the preceding claims, wherein in the first positional arrangement the camera (12) is deactivated, and wherein in the second positional arrangement the polymerization light source (11) is deactivated.

10. The dental light polymerization device of any of the preceding claims, wherein in the second positional arrangement an illumination light source (19) is activated.

11. The dental light polymerization device of claim 10, wherein the illumination light source (19) is configured to emit visible light having a spectrum comprising light at wavelengths between 380 nanometers and 750 nanometers.

12. The dental light polymerization device of claim 10, wherein the illumination light source (19) comprises a plurality of white LEDs.

13. The dental light polymerization device of any of the preceding claims, wherein the polymerization light source (19) is configured to emit visible light predominantly within a wavelength range 450 nanometers - 495 nanometers.

14. The dental light polymerization device of claim 13, wherein the polymerization light source (19) consists of a single high power LED.

## Patentansprüche

1. Eine zahnärztliche Lichtpolymerisierungsvorrichtung (1), umfassend eine Polymerisierungslichtquelle (11) und eine Kamera (12), wobei die Vorrichtung einen aktiv bewegbaren Lichtreflektor umfasst, **dadurch gekennzeichnet, dass** der Reflektor zwischen einer ersten Positionsanordnung (Fig. 1), in der der Reflektor einen ersten optischen Pfad (15) zwischen der Polymerisierungslichtquelle (11) und einem Objekt (500) herstellt, und einer zweiten Positionsanordnung, in der der Reflektor einen zweiten optischen Pfad (16) zwischen dem Objekt (500) und der Kamera (12) herstellt, bewegbar ist, wobei alternativ:
in der ersten Positionsanordnung der zweite optische Pfad (16) unterbrochen ist;
in der zweiten Positionsanordnung der erste optische Pfad (15) unterbrochen ist.

2. Die zahnärztliche Lichtpolymerisierungsvorrichtung nach Anspruch 1, wobei der Reflektor durch ein Prisma (21) gebildet ist.

3. Die zahnärztliche Lichtpolymerisierungsvorrichtung nach Anspruch 1, wobei der Reflektor durch einen Spiegel (13) gebildet ist.

4. Die zahnärztliche Lichtpolymerisierungsvorrichtung nach einem der vorstehenden Ansprüche, wobei der Reflektor zwischen der ersten und der zweiten Positionsanordnung drehbar ist.

5. Die zahnärztliche Lichtpolymerisierungsvorrichtung nach einem der vorstehenden Ansprüche, die betreibbar ist, um den Reflektor periodisch zwischen der ersten und der zweiten Positionsanordnung zu bewegen.

6. Die zahnärztliche Lichtpolymerisierungsvorrichtung nach Anspruch 5, wobei ein Zeitraum das Halten des Reflektors in der ersten Positionsanordnung für einen ersten Zeitraum, das Bewegen des Reflektors in die zweite Positionsanordnung, das Halten des Reflektors in der zweiten Positionsanordnung für einen zweiten Zeitraum und das Bewegen des Reflektors in die erste Positionsanordnung umfasst.

7. Die zahnärztliche Lichtpolymerisierungsvorrichtung nach Anspruch 6, wobei der erste und der zweite Zeitraum unterschiedlich sind, vorzugsweise in einem Verhältnis von etwa 40 : 1 bis etwa 2 : 1.

8. Die zahnärztliche Lichtpolymerisierungsvorrichtung nach Anspruch 3, wobei der Reflektor durch eine Vielzahl von Mikrospiegeln (13') gebildet ist, die durch eine digitale Mikrospiegelvorrichtung bereitgestellt sind.

9. Die zahnärztliche Lichtpolymerisierungsvorrichtung nach einem der vorstehenden Ansprüche, wobei in der ersten Positionsanordnung die Kamera (12) deaktiviert ist, und wobei in der zweiten Positionsanordnung die Polymerisierungslichtquelle (11) deaktiviert ist.

10. Die zahnärztliche Lichtpolymerisierungsvorrichtung nach einem der vorstehenden Ansprüche, wobei in der zweiten Positionsanordnung eine Beleuchtungslichtquelle (19) aktiviert ist.

11. Die zahnärztliche Lichtpolymerisierungsvorrichtung nach Anspruch 10, wobei die Beleuchtungslichtquelle (19) konfiguriert ist, um sichtbares Licht mit einem Spektrum, umfassend Licht bei Wellenlängen zwischen 380 Nanometern und 750 Nanometern, zu emittieren.

12. Die zahnärztliche Lichtpolymerisierungsvorrichtung nach Anspruch 10, wobei die Beleuchtungslichtquelle (19) eine Mehrzahl weißer LEDs umfasst.

13. Die zahnärztliche Lichtpolymerisierungsvorrichtung nach einem der vorstehenden Ansprüche, wobei die Polymerisierungslichtquelle (19) konfiguriert ist, um sichtbares Licht vorwiegend innerhalb eines Wellenlängenbereichs von 450 Nanometer - 495 Nanometer zu emittieren.

14. Die zahnärztliche Lichtpolymerisierungsvorrichtung nach Anspruch 13, wobei die Polymerisierungslichtquelle (19) aus einer einzelnen Hochleistungs-LED besteht.

## Revendications

1. Dispositif de polymérisation lumineuse dentaire (1), comprenant une source de lumière de polymérisation (11), et une caméra (12), dans lequel le dispositif comprend un réflecteur de lumière activement mobile,
**caractérisé en ce que** le réflecteur est mobile entre un premier agencement de position (Fig. 1), dans lequel le réflecteur établit un premier trajet optique (15) entre la source de lumière de polymérisation (11) et un objet (500), et un deuxième agencement de position, dans lequel le réflecteur établit un deuxième trajet optique (16) entre l'objet (500) et la caméra (12), dans lequel alternativement :
dans le premier agencement de position le deuxième trajet optique (16) est suspendu ;
dans le deuxième agencement de position le premier trajet optique (15) est suspendu.

2. Dispositif de polymérisation lumineuse dentaire selon la revendication 1, dans lequel le réflecteur est formé par un prisme (21).

3. Dispositif de polymérisation lumineuse dentaire selon la revendication 1, dans lequel le réflecteur est formé par un miroir (13).

4. Dispositif de polymérisation lumineuse dentaire selon l'une quelconque des revendications précédentes, dans lequel le réflecteur peut tourner entre le premier et le deuxième agencement de position.

5. Dispositif de polymérisation lumineuse dentaire selon l'une quelconque des revendications précédentes, étant opérationnel pour déplacer périodiquement le réflecteur entre le premier et le deuxième agencement de position.

6. Dispositif de polymérisation lumineuse dentaire selon la revendication 5, dans lequel une période comprend le maintien du réflecteur dans le premier agencement de position pendant une première période de temps, le déplacement du réflecteur au deuxième agencement de position, le maintien du réflecteur dans le deuxième agencement de position pendant une deuxième période de temps, et le déplacement du réflecteur au premier agencement de position.

7. Dispositif de polymérisation lumineuse dentaire selon la revendication 6, dans lequel les première et deuxième périodes de temps sont différentes, de préférence à un rapport d'environ 40:1 à environ 2:1.

8. Dispositif de polymérisation lumineuse dentaire selon la revendication 3, dans lequel le réflecteur est formé par une multiplicité de micromiroirs (13') fournis par un dispositif à micromiroirs numérique.

9. Dispositif de polymérisation lumineuse dentaire selon l'une quelconque des revendications précédentes, dans lequel dans le premier agencement de position la caméra (12) est désactivée, et dans lequel dans le deuxième agencement de position la source de lumière de polymérisation (11) est désactivée.

10. Dispositif de polymérisation lumineuse dentaire selon l'une quelconque des revendications précédentes, dans lequel dans le deuxième agencement de position une source de lumière d'éclairage (19) est activée.

11. Dispositif de polymérisation lumineuse dentaire selon la revendication 10, dans lequel la source de lumière d'éclairage (19) est configurée pour émettre de la lumière visible ayant un spectre comprenant de la lumière à des longueurs d'onde entre 380 nanomètres et 750 nanomètres.

12. Dispositif de polymérisation lumineuse dentaire selon la revendication 10, dans lequel la source de lumière d'éclairage (19) comprend une pluralité de DEL blanches.

13. Dispositif de polymérisation lumineuse dentaire selon l'une quelconque des revendications précédentes, dans lequel la source de lumière de polymérisation (19) est configurée pour émettre de la lumière visible principalement au sein d'une plage de longueur d'onde de 450 nanomètres à 495 nanomètres.

14. Dispositif de polymérisation lumineuse dentaire selon la revendication 13, dans lequel la source de lumière de polymérisation (19) est constituée d'une unique DEL à haute puissance.
